# EUROPEAN PATENT APPLICATION

(11) **EP 4 328 287 A1**
(43) Date of publication of application: **28.02.2024**
(21) Application number: 22842074.1
(22) Date of filing: 11.07.2022
(51) Int. Cl.: C10G 2/00, C10J 3/00, C25B 3/03, C25B 3/26

(54) **SYNTHETIC FUEL PRODUCTION METHOD**

(30) Priority: 12.07.2021 JP 2021115084
(71) Applicant: Toyo Engineering Corporation, Tokyo 105-0003 (JP)
(72) Inventor: KAMIYAMA, Keita, Narashino-shi, Chiba 275-0024 (JP)
(74) Representative: Gille Hrabal Partnerschaftsgesellschaft mbB Patentanwälte
(86) International application number: PCT/JP2022/027232
(87) International publication number: WO 2023/286730

(57) **Abstract**

An amount of atmospheric emission of carbon dioxide can be reduced by a method for producing a synthetic fuel including a gasification step G of gasifying waste by reacting it with oxygen and water at a high temperature, a carbon dioxide separation step S of separating carbon dioxide from a gasified gas G1 produced in the step G, an FT synthesis step FT of producing the synthetic fuel by Fischer-Tropsch synthesis from a synthetic gas G2 produced in the step S and a carbon dioxide electrolysis step E of electrolyzing the carbon dioxide separated in the step S to produce an electrolyzed gas G3 containing carbon monoxide and carbon dioxide, the electrolyzed gas G3 produced in the carbon dioxide electrolysis step E being supplied to the carbon dioxide separation step S such that carbon dioxide is separated from the gasified gas G1 and the electrolyzed gas G3.

## Description

### Field of the Invention

The present invention relates to a method for producing a synthetic fuel such as SAF (sustainable aviation fuel), a diesel fuel or the like from waste such as biomass or the like, and more specifically relates to a method for producing a synthetic fuel that can reduce an amount of atmospheric emission of carbon dioxide produced when gasifying waste by reacting the waste, oxygen and water at a high temperature.

### Background of the Invention

Conventionally, there has been known a technology of gasifying waste such as woody biomass, MSW (municipal solid waste) or the like by reacting the waste, oxygen and water in a gasification furnace at a high temperature, and from carbon monoxide and hydrogen thus obtained, producing a synthetic fuel by Fischer-Tropsch (FT) synthesis.

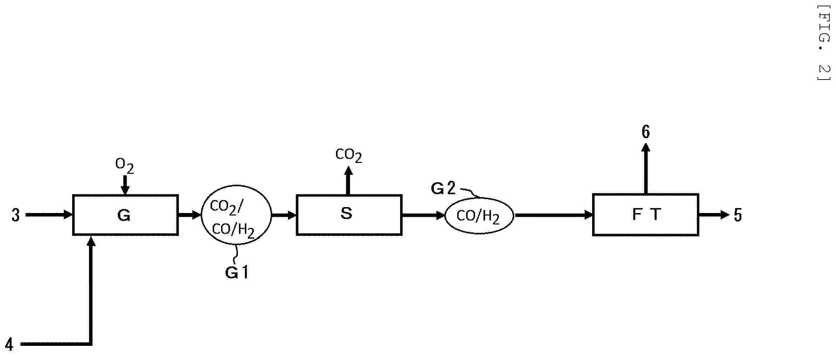

FIG. 2 is a diagram illustrating an example of a process flow of a conventional method for producing a synthetic fuel. This method illustrated in FIG. 2 includes a gasification step G of gasifying waste 3 such as woody biomass, MSW or the like by reacting the waste, oxygen and water 4 at a high temperature to produce a gasified gas G1 containing carbon dioxide, carbon monoxide and hydrogen, a carbon dioxide separation step S of separating carbon dioxide from the gasified gas G1 produced in the gasification step G and an FT synthesis step FT of producing a synthetic fuel by performing FT synthesis using a synthetic gas G2 (gas containing carbon monoxide and hydrogen) from which carbon dioxide has been separated in the carbon dioxide separation step S. In this conventional method, the carbon dioxide separated in the carbon dioxide separation step S is usually emitted into the atmosphere.

Note that examples of a method for producing a synthetic fuel using carbon dioxide as one of raw materials include a method described in patent literature 1. This patent literature 1 discloses a process including converting carbon dioxide and water to carbon monoxide and hydrogen by performing co-electrolysis in a syngas production cell (solid oxide electrolyzer cell), and then converting this to a hydrocarbon fuel in a catalytic reactor.

Main reactions when producing a synthetic fuel such as SAF or the like by FT synthesis from waste such as biomass or the like are expressed by the following reaction formulas:

CₚH_{q} + pH₂O -> pCO + (p + (q/2))H₂ (1)

CO + H₂O <--> CO₂ + H₂ (2)

nCO + (2n + 1)H₂ -> CₙH_{2n + 2} + nH₂O (3)

The above reaction formula (1) represents a reaction of producing carbon monoxide (CO) and hydrogen gas (H₂) by carrying out partial combustion or steam gasification of waste. Further, the reaction formula (3) represents a reaction of producing a synthetic fuel (CₙH_{2n + 2}) from carbon monoxide (CO) and hydrogen gas (H₂), where a use amount of hydrogen gas (H₂) is twice or more (2n + 1) a use amount (n) of carbon monoxide (CO). On the other hand, as represented by the reaction formula (2), a shift reaction occurs between carbon monoxide and water (CO + H₂O) and carbon dioxide and hydrogen gas (CO₂ + H₂). As a result, a production amount of hydrogen gas (H₂) decreases when a production amount of carbon monoxide (CO) increases, and in reverse, a production amount of carbon monoxide (CO) decreases when a production amount of hydrogen gas (H₂) increases. Therefore, when a production amount of hydrogen gas (H₂) is increased to twice or more a production amount of carbon monoxide, a production amount of carbon monoxide (CO) decreases for that. In addition, a production amount of carbon dioxide (CO₂) emitted into the atmosphere increases.

### Prior Art Document

### Patent Literature

Patent Literature 1: JP-A 2016-511296

### Summary of the Invention

### Problem to be solved by the Invention

In the conventional method illustrated in FIG. 2, the carbon dioxide separated in the carbon dioxide separation step S is usually emitted into the atmosphere. However, atmospheric emission of large amounts of carbon dioxide, which is one of the greenhouse gases, is not preferable from the viewpoint of preventing global warming. Therefore, in order to reduce an amount of atmospheric emission of carbon dioxide, the present inventor studied an effective method for recycling carbon dioxide.

In other words, an objective of the present invention is to provide a method for producing a synthetic fuel that can reduce an amount of atmospheric emission of carbon dioxide.

### Means to solve the Problem

As a result of diligent study to achieve the above objective, the present inventor found it very effective to combine a step such as a carbon dioxide electrolysis step or the like with the conventional method illustrated in FIG. 2 to recycle carbon monoxide produced by electrolysis as a raw material for FT synthesis, coming to complete the present invention. In other words, the present invention includes each aspect below.
[1] A method for producing a synthetic fuel including:
   a gasification step of gasifying waste by reacting the waste, oxygen and water at a high temperature to produce a gasified gas containing carbon dioxide, carbon monoxide and hydrogen;
   a carbon dioxide separation step of separating carbon dioxide at least from the gasified gas produced in the gasification step; and
   an FT synthesis step of producing the synthetic fuel by Fischer-Tropsch synthesis from a synthetic gas from which carbon dioxide has been separated in the carbon dioxide separation step,
   characterized in that the method for producing a synthetic fuel further includes a carbon dioxide electrolysis step of electrolyzing the carbon dioxide separated in the carbon dioxide separation step to produce an electrolyzed gas containing carbon monoxide and carbon dioxide,
   the electrolyzed gas produced in the carbon dioxide electrolysis step being supplied to the carbon dioxide separation step such that carbon dioxide is separated from the gasified gas and the electrolyzed gas.
[2] The method for producing a synthetic fuel according to [1], further including a water electrolysis step of electrolyzing water to produce oxygen and hydrogen, the produced hydrogen being supplied to the FT synthesis step and the produced oxygen being supplied to the gasification step.
[3] The method for producing a synthetic fuel according to [1] or [2], further including an oxygen separation step of separating oxygen from air, the separated oxygen being supplied to the gasification step.
[4] A method for improving a synthetic fuel production facility to reduce an amount of atmospheric emission of carbon dioxide generated in an apparatus of an existing synthetic fuel production facility,
   the existing synthetic fuel production facility including:
   a gasification apparatus for gasifying waste by reacting the waste, oxygen and water at a high temperature to produce a gasified gas containing carbon dioxide, carbon monoxide and hydrogen;
   a carbon dioxide separation apparatus for separating carbon dioxide at least from the gasified gas produced in the gasification apparatus; and
   an FT synthesis apparatus for producing a synthetic fuel by Fischer-Tropsch synthesis from a synthetic gas from which carbon dioxide has been separated in the carbon dioxide separation apparatus,
   the improvement method being characterized by adding to the facility, a carbon dioxide electrolysis apparatus for electrolyzing the carbon dioxide separated in the carbon dioxide separation apparatus to produce an electrolyzed gas containing carbon monoxide and carbon dioxide,
   the electrolyzed gas produced in the carbon dioxide electrolysis apparatus being supplied to the carbon dioxide separation apparatus such that carbon dioxide is separated from the gasified gas and the electrolyzed gas.
[5] The method for improving a synthetic fuel production facility according to [4], further adding a water electrolysis apparatus for electrolyzing water to produce oxygen and hydrogen, the produced hydrogen being supplied to the FT synthesis apparatus and the produced oxygen being supplied to the gasification apparatus.

### Effect of the Invention

In the present invention, an amount of atmospheric emission of carbon dioxide can be reduced as carbon dioxide emitted into the atmosphere in the conventional method is partially reduced to carbon monoxide in the carbon dioxide electrolysis step and this is recycled as a raw material for FT synthesis.

Further, hydrogen produced by electrolyzing water is preferably supplied to the FT synthesis step. This can supplement an insufficient amount of hydrogen in the raw material gas in the FT synthesis step. In this case, a molar amount of the hydrogen produced by electrolyzing water and supplied to the FT synthesis step is preferably twice or more a molar amount of carbon monoxide in the electrolyzed gas. This achieves a suitable compositional balance in the raw material gas in the FT synthesis step.

Further, it is also preferable to supply oxygen produced by electrolyzing water to the gasification step. Utilizing this oxygen for gasifying waste can reduce load on the oxygen separation step.

### Brief Description of the Drawings

[FIG. 1] A diagram illustrating an example of a process flow of a method for producing a synthetic fuel of the present invention.
[FIG. 2] A diagram illustrating an example of a process flow of a conventional method for producing a synthetic fuel.

### Embodiments of the Invention

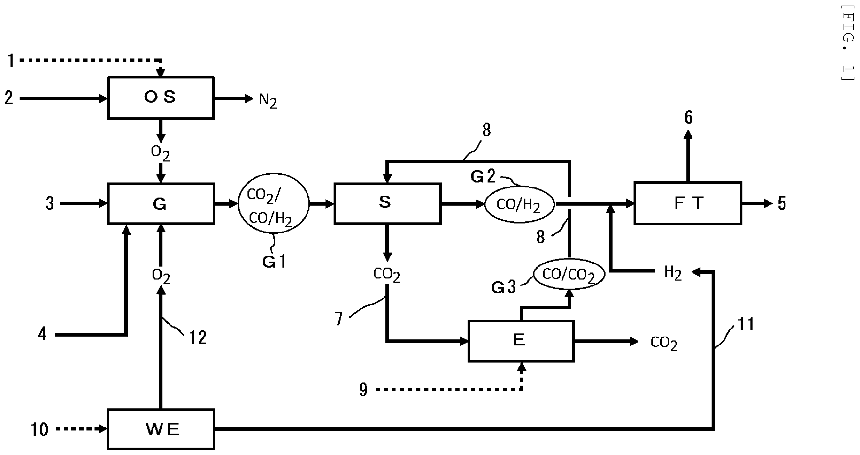

FIG. 1 is a diagram illustrating a process flow in a preferable form of a method for producing a synthetic fuel of the present invention. Each step is explained below. Supply of a material or electric power to each step can be carried out by providing as necessary a supply system including a supply line, and emission or removal of a product or waste from each step can be carried out by providing as necessary an emission system including an emission line.

### [Oxygen separation step]

An oxygen separation step OS shown in FIG. 1 is a step of separating oxygen from air 2. The oxygen separated in this oxygen separation step is supplied to a gasification step G described later.

Typical examples of a method for separating oxygen from air in this oxygen separation step include a method in which gases other than oxygen in the air (nitrogen or the like) are adsorbed onto adsorbents (for example, synthetic zeolite) by adjusting pressure to obtain a high-purity oxygen gas (vacuum pressure swing adsorption (VPSA) method). The adsorbed gases other than oxygen (nitrogen or the like) may be emitted into the atmosphere. As specific reaction conditions, types of adsorbents or configurations of reaction apparatuses including an oxygen separation apparatus or an oxygen separation system for that, publicly-known conditions, types and configurations relating to oxygen separation technology can be employed without any limitation.

In the present invention, it is preferable to use a VPSA process as above as one of steps for obtaining oxygen to be supplied to the gasification step G. However, the present invention is not limited thereto. It is also possible to obtain a high-purity oxygen gas by any other publicly-known methods (cryogenic separation method or the like) instead of a VPSA process and supply it to the gasification step G. It is often the case that a VPSA process is advantageous in terms of economy, but in a small plant, for example, a cryogenic separation method may be more advantageous in terms of economy.

### [Gasification step]

The gasification step G shown in FIG. 1 is a step of gasifying waste 3 by reacting the waste, water 4 and oxygen at a high temperature to produce a gasified gas G1 [CO₂/CO/H₂] containing carbon dioxide, carbon monoxide and hydrogen. The gasified gas G1 [CO₂/CO/H₂] produced in this gasification step G is supplied to a carbon dioxide separation step S described later.

Typical examples of a method for gasifying waste by reacting the waste, oxygen and water at a high temperature in this step G include a method in which the waste, oxygen and water are supplied to a gasification furnace (melting furnace) and reacted at predetermined temperature and pressure. As specific reaction conditions or configurations of reaction apparatuses including a gasification apparatus or a gasification system for that, publicly-known conditions and configurations relating to gasification technology can be employed without any limitation. For example, a reaction temperature is usually 700°C or more and preferably 800°C to 1200°C.

Examples of the waste used as a raw material in the gasification step G can include a solid and a liquid containing a gasifiable hydrocarbon component and a mixture of them, and an example is woody biomass or MSW (municipal solid waste). However, the present invention is not limited thereto. For example, waste such as herbaceous biomass, PKS (palm kernel shell), OPT (oil palm trunk) or the like can also be used.

### [Carbon dioxide separation step]

The carbon dioxide separation step S shown in FIG. 1 is a step of separating carbon dioxide from a gas of a treatment target which at least contains the gasified gas G1 [CO₂/CO/H₂]. As the gas of a treatment target, the gasified gas G1 alone or both the gasified gas G1 and an electrolyzed gas G3 [CO₂/CO] produced in a carbon dioxide electrolysis step E described later can be used. When carbon dioxide is separated from the gasified gas G1 and the electrolyzed gas G3, they may be mixed as necessary and supplied to the carbon dioxide separation step S.

The carbon dioxide separated in this carbon dioxide separation step S is not emitted into the atmosphere, but supplied to the carbon dioxide electrolysis step E described later and recycled via supply systems 7 and 8. As a result, an amount of atmospheric emission of carbon dioxide can be reduced.

On the other hand, a gas after carbon dioxide has been separated, in other words, a synthetic gas G2 [CO/H₂] containing carbon monoxide and hydrogen is supplied to an FT synthesis step FT described later as a raw material of a synthetic fuel.

Typical examples of a method for separating carbon dioxide from the gas of a treatment target in this step S include a chemical absorption method including absorbing carbon dioxide by an absorption liquid such as an amine or the like in an absorption step and heating the absorption liquid to separate carbon dioxide in a regeneration step. As specific reaction conditions or configurations of reaction apparatuses including a carbon dioxide separation apparatus or a carbon dioxide separation system for that, publicly-known conditions and configurations relating to carbon dioxide separation technology can be employed without any limitation.

### [Carbon dioxide electrolysis step]

The carbon dioxide electrolysis step E shown in FIG. 1 is a step of electrolyzing the carbon dioxide separated in the carbon dioxide separation step S to produce an electrolyzed gas G3 [CO/CO₂] containing carbon monoxide and carbon dioxide. The electrolyzed gas G3 [CO/CO₂] produced in this carbon dioxide electrolysis step E is returned to the carbon dioxide separation step S.

The carbon dioxide electrolysis step E is typically a step of partially reducing carbon dioxide to carbon monoxide by electrolysis. Accordingly, the produced electrolyzed gas G3 [CO/CO₂] is typically a mixed gas of carbon monoxide produced by reduction and unreduced carbon dioxide. As specific electrolysis conditions or configuration of electrolysis apparatus or electrolysis system for that, publicly-known conditions and configuration relating to carbon dioxide electrolysis technology can be employed without any limitation.

This step of partially reducing carbon dioxide to carbon monoxide by electrolysis also has the advantages of allowing electrolysis at a low temperature (less than 100°C) and also not causing the problem of performance degradation due to adhesion of deposited carbon to an electrode compared to a method as described in patent literature 1 (a process in which carbon dioxide and water are converted to carbon monoxide and hydrogen by performing co-electrolysis at a high temperature (500°C or more) in a solid oxide electrolyzer cell).

Further, as the electrolyzed gas G3 produced in the carbon dioxide electrolysis step E is returned to the carbon dioxide separation step S via the supply system 8, carbon monoxide in the electrolyzed gas G3 [CO/CO₂] will be part of the raw material of the synthetic fuel.

Note that the carbon dioxide after the treatment in the carbon dioxide electrolysis step E may be all supplied to the carbon dioxide separation step S, but some of the carbon dioxide is preferably emitted into the atmosphere. The reason for this is to prevent inert gases such as nitrogen or the like, which are impurities in oxygen supplied to the gasification step G, from accumulating within the system. However, an emission amount of carbon dioxide emitted into the atmosphere in this step is a very small amount compared to an emission amount in the conventional method illustrated in FIG. 2 (i.e., a total amount of carbon dioxide separated in the carbon dioxide separation step S). Therefore, the present invention can reduce an emission amount of carbon dioxide sufficiently compared to the conventional method.

It is preferable to use electric power 9 generated by renewable energy for the carbon dioxide electrolysis step E. Renewable energy is energy always present in the natural world such as sunlight, wind power, geothermal heat, water power or the like, and characterized by not emitting carbon dioxide when generated. Using electric power from this renewable energy for the carbon dioxide electrolysis step E agrees with the objective of the present invention to reduce an emission amount of carbon dioxide.

### [Water electrolysis step]

A water electrolysis step WE shown in FIG. 1 is a step of electrolyzing water to produce oxygen and hydrogen. As specific electrolysis conditions or configuration of electrolysis apparatus or electrolysis system in this step WE, publicly-known conditions and configuration relating to water electrolysis technology can be employed without any limitation.

In the water electrolysis step WE, the produced hydrogen is supplied to the FT synthesis step FT described later via a supply system 11. When an amount of hydrogen in the raw material gas in the FT synthesis step FT is insufficient, this insufficient amount of hydrogen can be supplemented with this. A molar amount of the hydrogen produced in the water electrolysis step WE and supplied to the FT synthesis step is preferably twice or more a molar amount of carbon monoxide in the electrolyzed gas G3. This achieves a suitable compositional balance in the raw material gas in the FT synthesis step FT.

On the other hand, the oxygen produced in the water electrolysis step WE is supplied to the gasification step G via a supply system 12. Utilizing this oxygen for gasifying waste can reduce load on the oxygen separation step OS.

It is preferable to use electric power 10 generated by renewable energy for the water electrolysis step WE as is the case with the carbon dioxide electrolysis step E explained above.

In the present invention, it is preferable to use the water electrolysis step WE explained above as one of steps for producing hydrogen to be supplied to the FT synthesis step FT. However, the present invention is not limited thereto. It is also possible to produce hydrogen by any other publicly-known methods instead of the water electrolysis step WE and supply it to the FT synthesis step FT.

### [FT synthesis step]

The FT synthesis step FT shown in FIG. 1 is a step of producing a synthetic fuel by Fischer-Tropsch (FT) synthesis from the synthetic gas G2 from which carbon dioxide has been separated in the carbon dioxide separation step S, in other words, the synthetic gas G2 [CO/H₂] containing carbon monoxide and hydrogen.

Fischer-Tropsch (FT) synthesis is a synthesis method for obtaining a synthetic fuel (gas and liquid hydrocarbon) from carbon monoxide and hydrogen by a catalytic reaction. A compound of iron or cobalt is usually used as a catalyst. As specific reaction conditions, types of catalysts or configurations of reaction apparatuses including an FT synthesis apparatus or an FT synthesis system in this FT synthesis, publicly-known conditions, types and configurations relating to FT synthesis technology can be employed without any limitation.

This FT synthesis step FT produces SAF (sustainable aviation fuel) and other synthetic fuels 5. Examples of the other synthetic fuels include, for example, kerosene, diesel oil, naphtha or the like. Further, a gas fraction produced at the time of synthesis is used as a fuel gas or burned by flare or the like and released into the atmosphere as an off-gas 6.

### [Method for improving synthetic fuel production facility]

The method for producing a synthetic fuel of the present invention explained above can be carried out by newly constructing all apparatuses that each carry out each of the steps. However, it can be carried out also by adding a carbon dioxide electrolysis apparatus and as necessary other apparatuses (for example, water electrolysis apparatus) to an existing production facility.

In other words, a method for improving a synthetic fuel production facility of the present invention is an improvement method for reducing an amount of atmospheric emission of carbon dioxide generated in an apparatus of an existing synthetic fuel production facility.

Examples of the existing synthetic fuel production facility as a target to be improved can include, as one example, an existing synthetic fuel production facility including a gasification apparatus (g) for gasifying waste by reacting the waste, oxygen and water at a high temperature to produce the gasified gas G1 containing carbon dioxide, carbon monoxide and hydrogen, a carbon dioxide separation apparatus (s) for separating carbon dioxide at least from the gasified gas G1 produced in the gasification apparatus (g) and an FT synthesis apparatus for producing a synthetic fuel by Fischer-Tropsch synthesis from the synthetic gas G2 from which carbon dioxide has been separated in the carbon dioxide separation apparatus (s), the facility releasing the carbon dioxide separated in the carbon dioxide separation apparatus (s) into the atmosphere.

In the improvement method of the present invention, a carbon dioxide electrolysis apparatus (e) for electrolyzing carbon dioxide to produce the electrolyzed gas G3 containing carbon monoxide and carbon dioxide, the supply system 7 for supplying the carbon dioxide generated in the carbon dioxide separation apparatus (s) to the carbon dioxide electrolysis apparatus (e) and the supply system 8 for supplying the electrolyzed gas G3 produced in the carbon dioxide electrolysis apparatus (e) to the carbon dioxide separation apparatus (s) are added to the existing synthetic fuel production apparatus.

This addition of the carbon dioxide electrolysis apparatus (e) and a circulation system including the supply systems 7 and 8 enables an effective reduction in an emission amount of carbon dioxide as carbon dioxide released into the atmosphere from the carbon dioxide separation apparatus (s) in the existing facility is collected to the carbon dioxide electrolysis apparatus (e) and partially converted in the carbon dioxide electrolysis apparatus (e) to carbon monoxide, which is utilized as a component of the synthetic gas G2.

Further, in this improvement method, it is preferable to add a water electrolysis apparatus (we) for electrolyzing water to produce oxygen and hydrogen and supply the produced hydrogen to the FT synthesis apparatus via the supply system 11. In this case, a molar amount of the hydrogen produced in the water electrolysis apparatus (we) and supplied to the FT synthesis apparatus is preferably twice or more a molar amount of carbon monoxide in the electrolyzed gas G3. Further, it is also preferable to supply the oxygen produced in the water electrolysis apparatus (we) to the gasification apparatus (g).

Adding an apparatus such as the carbon dioxide electrolysis apparatus or the like to an existing production facility in this manner is advantageous in terms of facility costs compared to when newly constructing all facilities. Further, a production amount of a synthetic fuel can be increased by effectively utilizing carbon dioxide emitted from an existing production facility.

### Industrial Applicability

The present invention is very useful from the viewpoint of preventing global warming as it can reduce an amount of atmospheric emission of carbon dioxide by recycling carbon dioxide produced when producing a synthetic fuel from waste.

### Description of the Reference Numerals

1: electric power
2: air
3: waste
4: water
5: fuel
6: off-gas
7: carbon dioxide supply system
8: electrolyzed gas supply system
9: electric power generated by renewable energy
10: electric power generated by renewable energy
11: hydrogen supply system
12: oxygen supply system
OS: oxygen separation step
G: gasification step
S: carbon dioxide separation step
FT: FT synthesis step
E: carbon dioxide electrolysis step
WE: water electrolysis step
G1: gasified gas
G2: synthetic gas
G3: electrolyzed gas

## Claims

1. A method for producing a synthetic fuel comprising:
a gasification step of gasifying waste by reacting the waste, oxygen and water at a high temperature to produce a gasified gas containing carbon dioxide, carbon monoxide and hydrogen;
a carbon dioxide separation step of separating carbon dioxide at least from the gasified gas produced in the gasification step; and
an FT synthesis step of producing the synthetic fuel by Fischer-Tropsch synthesis from a synthetic gas from which carbon dioxide has been separated in the carbon dioxide separation step,
**characterized in that** the method for producing a synthetic fuel further comprises a carbon dioxide electrolysis step of electrolyzing the carbon dioxide separated in the carbon dioxide separation step to produce an electrolyzed gas containing carbon monoxide and carbon dioxide,
the electrolyzed gas produced in the carbon dioxide electrolysis step being supplied to the carbon dioxide separation step such that carbon dioxide is separated from the gasified gas and the electrolyzed gas.

2. The method for producing a synthetic fuel according to claim 1, further comprising a water electrolysis step of electrolyzing water to produce oxygen and hydrogen, the produced hydrogen being supplied to the FT synthesis step and the produced oxygen being supplied to the gasification step.

3. The method for producing a synthetic fuel according to claim 1 or 2, further comprising an oxygen separation step of separating oxygen from air, the separated oxygen being supplied to the gasification step.

4. A method for improving a synthetic fuel production facility to reduce an amount of atmospheric emission of carbon dioxide generated in an apparatus of an existing synthetic fuel production facility,
the existing synthetic fuel production facility comprising:
a gasification apparatus for gasifying waste by reacting the waste, oxygen and water at a high temperature to produce a gasified gas containing carbon dioxide, carbon monoxide and hydrogen;
a carbon dioxide separation apparatus for separating carbon dioxide at least from the gasified gas produced in the gasification apparatus; and
an FT synthesis apparatus for producing a synthetic fuel by Fischer-Tropsch synthesis from a synthetic gas from which carbon dioxide has been separated in the carbon dioxide separation apparatus,
the improvement method being **characterized by** adding to the facility, a carbon dioxide electrolysis apparatus for electrolyzing the carbon dioxide separated in the carbon dioxide separation apparatus to produce an electrolyzed gas containing carbon monoxide and carbon dioxide,
the electrolyzed gas produced in the carbon dioxide electrolysis apparatus being supplied to the carbon dioxide separation apparatus such that carbon dioxide is separated from the gasified gas and the electrolyzed gas.

5. The method for improving a synthetic fuel production facility according to claim 4, further adding a water electrolysis apparatus for electrolyzing water to produce oxygen and hydrogen, the produced hydrogen being supplied to the FT synthesis apparatus and the produced oxygen being supplied to the gasification apparatus.
